# EUROPEAN PATENT SPECIFICATION

(11) **EP 2 552 373 B1**
(45) Date of publication and mention of the grant of the patent: **09.11.2016**
(21) Application number: 11762928.7
(22) Date of filing: 31.03.2011
(51) Int. Cl.: A61F 13/15, A61F 13/472, A61F 13/56

(54) **Individually Wrapped Absorbent Product**
INDIVIDUELL GEWICKELTES SAUGFÄHIGES PRODUKT
PRODUIT ABSORBANT À EMBALLAGE INDIVIDUEL

(30) Priority: 31.03.2010 JP 2010084170
(43) Date of publication of application: 06.02.2013
(73) Proprietor: Unicharm Corporation, Ehime 799-0111 (JP)
(72) Inventor: NOMOTO, Takashi, Kanonji-shi Kagawa 769-1602 (JP); ONOZUKA, Takashi, Kanonji-shi Kagawa 769-1602 (JP); OBA, Kenji, Kanonji-shi Kagawa 769-1602 (JP)
(74) Representative: Peter, Julian
(86) International application number: PCT/JP2011/058753
(87) International publication number: WO 2011/122713

(56) References cited:
- EP-A1- 0 347 319
- WO-A1-99/60965
- WO-A1-2009/096402
- JP-A- 2006 141 943
- JP-B1- 4 558 080
- JP-B1- 4 558 081
- JP-B1- 4 558 082
- JP-U- 59 190 231
- JP-Y2- 6 047 459
- US-A- 5 788 686
- US-A1- 2006 206 077
- US-B1- 6 375 645

## Description

### Technical Field

The present invention relates to an individually wrapped absorbent product.

### Background Art

Known in the art is an absorbent product provided with an absorbent body which includes a liquid permeable skin contact side sheet, a liquid impermeable non-skin contact side sheet, and an absorbent core which is arranged between these skin contact side sheet and non-skin contact side sheet and with a cover sheet which covers a surface of the skin contact side sheet (see PLT 1). In this absorbent product, the skin contact side sheet surface is kept from becoming dirty before use by the cover sheet.

On the other hand, known in the art is an individually wrapped product formed by superposing a wrapping sheet over the absorbent product at the non-skin contact side sheet side, folding up the absorbent product and wrapping sheet so that the skin contact side sheet is positioned at the inside, and sealing the side edges of the wrapping sheet (see PLT 2). In this individually wrapped product, the absorbent product as a whole is covered by the wrapping sheet, so the surface of the skin contact side sheet is protected from the outside. Further, a wrapping sheet is interposed between the surface of the skin contact side sheet and the adhesive strips of the absorbent product, so the surface of the skin contact side sheet is protected from the adhesive strips.

### Citation List

### Patent Literature

PLT 1: Japanese Patent No. 3163233
PLT 2: Japanese Patent Publication (A) No. 9-512454

### Summary of Invention

### Technical Problem

However, PLT 1 does not disclose at all how to individually wrap an absorbent product having a cover sheet.

Further, what PLT 2 discloses is an individually wrapped absorbent product not having a cover sheet, so PLT 2 cannot be applied to PLT 1 as it is. That is to say, in PLT 2, the surface of the skin contact side sheet is exposed, so it is necessary to protect the surface of the skin contact side sheet by the wrapping sheet, while in PLT 1, the surface of the skin contact side sheet is covered by the cover sheet. That is, when forming an individually wrapped product, the situation completely differs between an individually wrapped absorbent product which has a cover sheet and an individually wrapped absorbent product which does not have a cover sheet.

Furthermore, in PLT 2, a wrapping sheet larger than the absorbent product is required. This is liable to result in a larger cost becoming necessary for forming an individually wrapped product.

### Solution to Problem

According to the present invention, there is provided an individually wrapped absorbent product provided with an absorbent body which includes a liquid permeable skin contact side sheet, a liquid impermeable non-skin contact side sheet, and an absorbent core which is arranged between these skin contact side sheet and non-skin contact side sheet and with a cover sheet which covers the surface of the skin contact side sheet, which individually wrapped product is formed by wrapping an absorbent product in a state to be wrapped, defined as a "ready-to-wrap product", by a wrapping material, in the individually wrapped absorbent product, the wrapping material including a belt-shaped material covering the ready-to-wrap product over the entire circumference of the ready-to-wrap product, the width of the belt-shaped material being shorter than a length of the ready-to-wrap product in a width direction of the belt-shaped material.

### Advantageous Effect of Invention

It is possible to provide an individually wrapped product suitable for an absorbent product which has a cover sheet.

### Brief Description of Drawings

FIG. 1 is a front view of a sanitary napkin.
FIG. 2 is a schematic lateral cross-sectional view of a sanitary napkin seen along the line II-II of FIG. 1.
FIG. 3 is a front view of the sanitary napkin of FIG. 1 with the wings opened up.
FIGS. 4A and 4B give schematic views for explaining a ready-to-wrap napkin.
FIGS. 5A, 5B and 5C give a schematic plan view and schematic side views of an individually wrapped product.
FIGS. 6A and 6B give a schematic plan view and a schematic side view of an individually wrapped product showing another embodiment according to the present invention.
FIG. 7 is a schematic side view of an individually wrapped product showing another embodiment of a wrapping material.
FIGS. 8A and 8B give a schematic front view and a schematic back view showing still another embodiment according to the present invention.

### Description of Embodiments

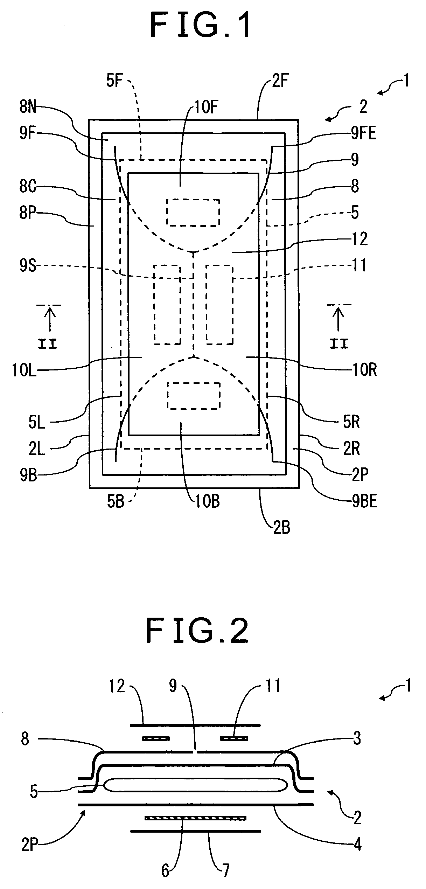

FIG. 1 and FIG. 2 show a case of application of the present invention to a sanitary napkin. However, the present invention can also be applied to another absorbent product such as a panty liner or incontinence pad.

Referring to FIG. 1 and FIG. 2, a sanitary napkin 1 of an embodiment according to the present invention (hereinafter simply referred to as a "napkin") is provided with an absorbent body 2. This absorbent body 2 is provided with a liquid permeable skin contact side sheet 3, a liquid impermeable non-skin contact side sheet 4, and a liquid holding absorbent core 5 arranged between these skin contact side sheet 3 and non-skin contact side sheet 4. The skin contact side sheet 3 and non-skin contact side sheet 4 are substantially the same size and are bonded with each other at the peripheral edge part 2P by a hot melt adhesive, heat sealing, etc. Further, at the outer surface of the non-skin contact side sheet 4 which faces clothing such as an undergarment at the time of use, adhesive strips 6 are applied for fastening the absorbent body 2 to the clothing. The adhesive strips 6 are covered by a peeloff sheet 7.

Note that, 2F, 2B, 2L, and 2R indicate a front end edge, back end edge, left side edge, and right side edge of the napkin 1 or absorbent body 2, while 5F, 5B, 5L, and 5R indicate a front end edge, back end edge, left side edge, and right side edge of the absorbent core 5. The front, back, left, and right in these cases correspond to the front, back, left, and right of the body of the wearer.

The surface of the skin contact side sheet 3 which contacts the skin of the wearer at the time of use is covered by a liquid impermeable cover sheet 8. This cover sheet 8 is substantially the same size as the absorbent body 2 and, therefore, covers substantially the entire surface of the skin contact side sheet 3. Further, the cover sheet 8 is bonded at the ring-shaped peripheral edge region 8P by a hot melt adhesive, heat sealing, etc. to the skin contact side sheet 3 or the absorbent body 2. As opposed to this, the region other than the peripheral edge region 8P, that is, the center region 8C, is not bonded to the skin contact side sheet 3. Alternatively, the cover sheet 8 may be a liquid permeable.

At the center region 8C of the cover sheet 8, partition lines 9 are formed in advance. These partition lines 9 are used to define or form at least one wing at the center region 8C for fastening the absorbent body 2 to the clothing. The partition lines 9 are comprised of cut lines completely cutting the cover sheet 8. Note that, the partition lines 9 can also be comprised of weakened lines such as perforated lines.

In an embodiment according to the present invention, the partition lines 9 are comprised of a U-shaped curved part 9F to be extended toward the front end edge 2F, a U-shaped curved part 9B to be extended toward the back end edge 2B, and a straight part 9S connecting the vertexes of these curved parts 9F and 9B at the substantial center of the cover sheet 8. As a result, at the center region 8C, four wings, that is, a semi oval front wing 10F and back wing 10B and a plateau-shaped left wing 10L and right wing 10R are defined. Here, the front wing 10F and back wing 10B are positioned symmetrically and the left and right wings 10L and 10R are positioned symmetrically.

In this case, the partition lines 9 do not reach the peripheral edge region 8P, therefore a non-cut region 8N is formed between the ends of the partition lines 9 and the peripheral edge region 8P.

Further, the partition lines 9 are formed so that the ends 9FE of the front side partition line part 9F are positioned at the outside in the longitudinal direction from the front end edge 5F of the absorbent core 5, while the ends 9BE of the back side partition line part 9B are positioned at the inside in the longitudinal direction from the back end edge 5B of the absorbent core 5.

At the outside surfaces of these wings 10F, 10B, 10L, and 10R, adhesive strips 11 are applied for fastening the corresponding wings 10F, 10B, 10L, and 10R to the clothing. Further, these adhesive strips 11 are covered by a common peeloff sheet 12.

Next, the materials of the different elements will be explained.

The skin contact side sheet 3 is for example comprised of a nonwoven fabric having holes or not having holes or a porous plastic sheet.

The non-skin contact side sheet 4 is for example comprised of a hydrophobic nonwoven fabric, a water impermeable plastic film, a laminate sheet of a nonwoven fabric and a water impermeable plastic film, a highly water resistant melt blown nonwoven fabric, or an SMS nonwoven fabric sandwiched between high strength spun bond nonwoven fabrics.

The absorbent core 5 is for example comprised of a fluffy pulp or air laid nonwoven fabric and highly absorbent polymer. Here, the fluffy pulp is for example comprised of chemical pulp, cellulose fiber, rayon, acetate, or other manmade cellulose fiber, while the air laid nonwoven fabric is for example comprised of pulp and synthetic fiber hot bonded or bonded by a binder to form a nonwoven fabric. The highly absorbent polymer is for example comprised of a starch-based, acrylic acid-based, or amino acid-based polymer of a particle or fiber shape.

The adhesive strips 6 and 11 are for example comprised of a styrene-isoprene-styrene block copolymer (SIS), styrene-butadiene-styrene block copolymer (SBS), styrene-ethylene-butylene-ethylene copolymer (SEBS), or other hot melt adhesive.

The cover sheet 8 is for example comprised of a hydrophobic nonwoven fabric, a water impermeable plastic film, a laminate sheet of a nonwoven fabric and a water impermeable plastic film, a highly water resistant melt blown nonwoven fabric, or an SMS nonwoven fabric sandwiched between high strength spun bond nonwoven fabrics, preferably a hydrophobic nonwoven fabric. The cover sheet 8 preferably has a basis weight of 15 g/m² to 60 g/m². When comprised of a plastic film, the plastic film preferably has a drapeability, by the cantilever method, of 20 mm to 100 mm, more preferably 30 mm to 70 mm. This is because if the drapeability is smaller than 20 mm, the cover sheet 8 will easily become twisted. On the other hand, if the drapeability is larger than 100 mm, the cover sheet 8 will become hard, so the feeling of use of the napkin 1 is liable to be degraded, the ability to mold against the clothing falls, and the liquid to be absorbed is liable to leak.

Note that, the above-mentioned cantilever method is performed as follows based on JIS-L1018. That is, five measurement pieces of a length of 150 mm and a width of 25 mm are stacked to obtain a measurement sample. Next, a cantilever made by Daiei Kagaku Seiki is used, the measurement sample is placed under a holding plate of the cantilever, the sample is slid in the slanted direction, and the distance of movement is automatically measured at a speed of 5 mm/sec. The measurement is performed for both the case where the front surface of the measurement sample is placed facing down and the case where the back surface of the measurement sample is placed facing down and the average of these was made the measurement result.

Now, the napkin 1 is fastened to the clothing as follows. That is, first, the peeloff sheet 7 at the non-skin contact side sheet 4 side is taken off and the napkin 1 or absorbent body 2 is fastened to the clothing through the adhesive strips 6. At this time, the skin contact side sheet 3 is covered by the cover sheet 8, so the skin contact side sheet 3 is prevented from becoming dirty.

Next, the peeloff sheet 12 of the cover sheet 8 side is taken off. As a result, four separate wings 10F, 10B, 10L, and 10R are formed.

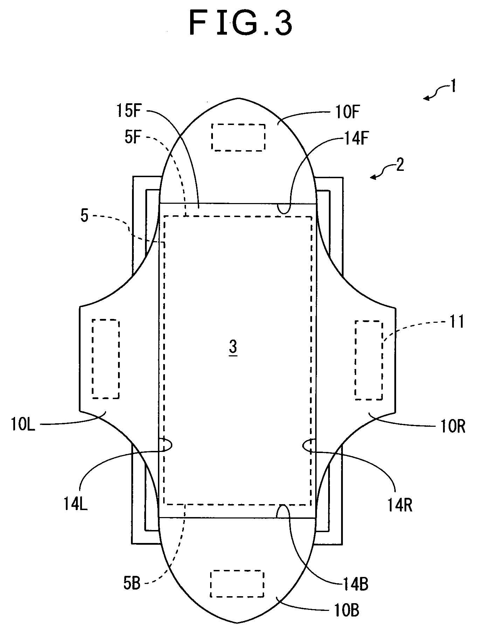

Next, as shown in FIG. 3, these wings 10F, 10B, 10L, and 10R are opened up. That is, the front wing 10F is folded at the front folded region 14F along the front end edge 2F and extended in the front direction, while the back wing 10B is folded at the back folded region 14B along the back end edge 2B and extended in the back direction. Further, the left wing 10L is folded at the left folded region 14L along the left side edge 2L and extended in the left direction, while the right wing 10R is folded at the right folded region 14R along the right side edge 2R and extended in the right direction. As a result, the skin contact side sheet 3 is exposed.

Next, the wings 10F, 10B, 10L, and 10R are fastened through the adhesive strips 11 to the clothing. That is, the front wing 10F is fastened to the inside surface of the clothing at the front of the absorbent body 2, while the back wing 10B is fastened to the inside surface of the clothing at the back of the absorbent body 2. Further, the left wing 10L and right wing 10R are fastened to the outside surface of the clothing at the bottom of the absorbent body 2.

As a result, even if external force acts on the absorbent body 2 from various directions, it is possible to suppress twisting or offset of position of the absorbent body 2. That is, it is possible to reliably fasten the absorbent body 2 to the clothing, therefore it is possible to suppress leakage of the liquid to be absorbed. Further, it is possible to eliminate the concern of the wearer over leakage.

Note that, the peripheral edge region 8P preferably has a width of 2 mm to 10 mm, more preferably 3 mm to 5 mm. This is because if the peripheral edge region 8P has a width narrower than 2 mm, the bonding strength between the cover sheet 8 and the absorbent body 2 will fall. Further, if the peripheral edge region 8P has a width broader than 10 mm, the surface of the skin contact side sheet 3 which is exposed when the wings 10F, 10B, 10L, and 10R are extended becomes narrower. That is, if the skin contact side sheet is broadly exposed, even if the positions of fastening the napkin 1 or absorbent body 2 to the clothing C are off somewhat from the suitable positions, leakage of the liquid to be absorbed can be suppressed.

Further, the size of the non-cut region 8N is preferably 2 mm to 10 mm, more preferably 3 mm to 5 mm. This is because even if the material meanders at the time of production of the napkin 1, the absorbent core 5 will not be positioned at the peripheral edge part 2P.

In an embodiment according to the present invention, an individually wrapped product is formed comprised of a napkin 1 which is individually wrapped. That is, a single napkin 1 is assembled into a ready-to-wrap state, the napkin in the ready-to-wrap state, defined as a "ready-to-wrap napkin", is wrapped by the wrapping material, and thereby an individually wrapped product is formed.

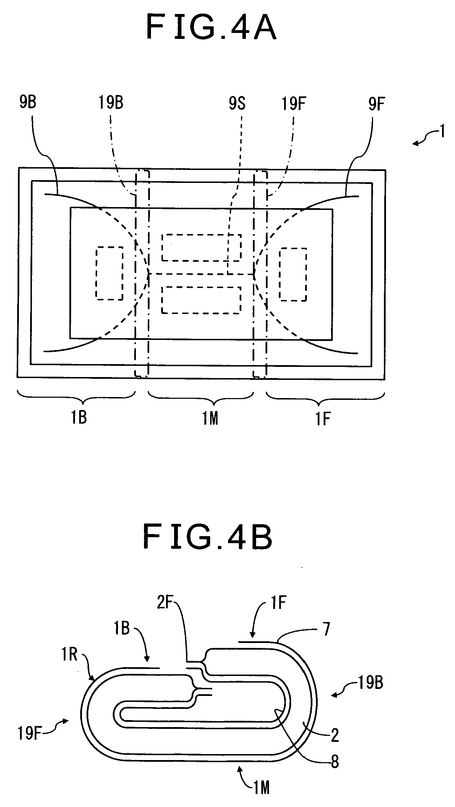

The ready-to-wrap napkin is for example formed as follows. That is, in the example shown in FIGS. 4A and 4B, a napkin 1 is folded along two folding regions 19F and 19B, which extend in the width direction of the napkin 1, so that the cover sheet 8 is positioned at the inside. Specifically, the napkin 1 is folded along the folding region 19B so that a back part 1B at the back from the folding region 19B is superposed over a middle part 1M between the folding region 19F and the folding region 19B. Next, the napkin 1 is folded along the folding region 19F so that a front part 1F at the front from the folding region 19F is superposed over the back part 1B. In this way, a ready-to-wrap napkin 1R is formed.

In this case, the front end edge 2F of the napkin 1 is superposed over the remaining part of the napkin 1. That is, the napkin 1 is folded so that one end of the napkin 1 in the longitudinal direction is superposed over the remaining part of the napkin 1.

Next, the ready-to-wrap napkin 1R is wrapped by the wrapping material whereby an individually wrapped product 1W is formed.

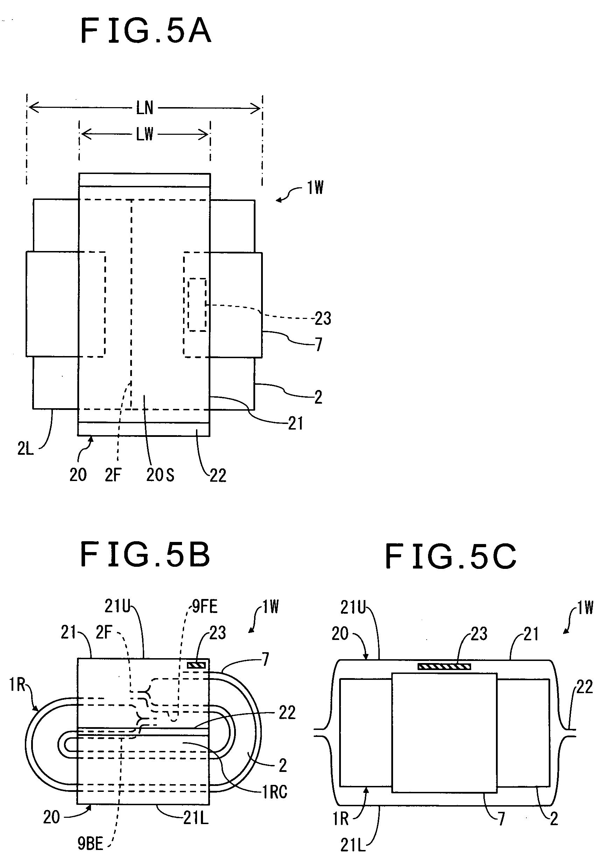

That is, as shown in FIGS. 5A, 5B and 5C, the wrapping material 20 includes a belt-shaped material 21 which surrounds the ready-to-wrap napkin 1R across the entire circumference of the ready-to-wrap napkin 1R. In this case, the belt-shaped material 21 is provided with an upper piece 21U and a lower piece 21L. These pieces 21U and 21L are bonded together at a bonding region 22 by for example a hot melt adhesive or hot sealing.

In this case, the width LW of the belt-shaped material 21 is made shorter than the length LN of the ready-to-wrap napkin 1R in the width LW direction. As a result, the wrapping material 20 partially wraps the ready-to-wrap napkin 1R, that is, does not wrap the ready-to-wrap napkin 1R as a whole, so it is possible to reduce the amount of the wrapping material 20.

Even if the wrapping of the ready-to-wrap napkin 1R is partial, the skin contact side sheet 3 is covered by the cover sheet 8 and, further, the napkin 1 is folded so that the cover sheet 8 is positioned at the inside, so the skin contact side sheet 3 is sufficiently protected.

Further, the belt-shaped material 21 is provided so as to extend in the width direction of the napkin 1, that is, is provided so as to extend cutting across the side edges 2L and 2R of the napkin 1. As a result, dirt etc. can be blocked from entering into clearances 1RC of the ready-to-wrap napkin 1R.

Furthermore, as shown in FIG. 5B, the belt-shaped material 21 is provided so as to extend crossing the ends 9FE and 9BE of the partition lines 9F and 9B. As a result, movement of the wings 10F, 10B, 10L, and 10R is suppressed. Therefore, the wings 10F, 10B, 10L, and 10R are kept from wrinkling. That is, if the wings 10F, 10B, 10L, and 10R wrinkle, the adhesive strips 11 applied to the wings 10F, 10B, 10L, and 10R are liable to end up sticking to other parts of the adhesive strips 11. In this embodiment according to the present invention, it is possible to deal with this inconvenience.

Furthermore, the belt-shaped material 21 is provided so as to extend across the front end edge 2F of the napkin 1. As a result, the ready-to-wrap napkin 1R can be kept from returning to its original flat shape and therefore the shape of the ready-to-wrap napkin 1R can be maintained.

Furthermore, an adhesive strip 23 is applied to the inside surface of the belt-shaped material 21. Through this adhesive strip 23, the belt-shaped material 21 is attached to the peeloff sheet 7. As a result, the belt-shaped material 21 can be prevented from shifting in position from the ready-to-wrap napkin 1R.

In this case, the belt-shaped material 21 is attached to the peeloff sheet 7 to be able to be peeled off from it. Of course, the belt-shaped material 21 may also be attached to the peeloff sheet piece 7 in an unpeelable manner, alternatively. Therefore, the wrapping material 20 is attached to the ready-to-wrap napkin 1R in a peelable or unpeelable manner.

The wearer removes the wrapping material 20 or belt-shaped material 21 from the ready-to-wrap napkin 1 and opens up the folded ready-to-wrap napkin 1R for use. In this case, since the ready-to-wrap napkin 1R is formed so that the cover sheet 8 is positioned at the inside, when opening up the ready-to-wrap napkin 1R, the napkin 1 tends to bulge outward with the non-skin contact side sheet 4 at the outside. As a result, the napkin 1 can be made to fit closer to the wearer.

Note that, the outer surface 20S of the wrapping material 20 may have letters, patterns, etc. printed or otherwise provided on it.

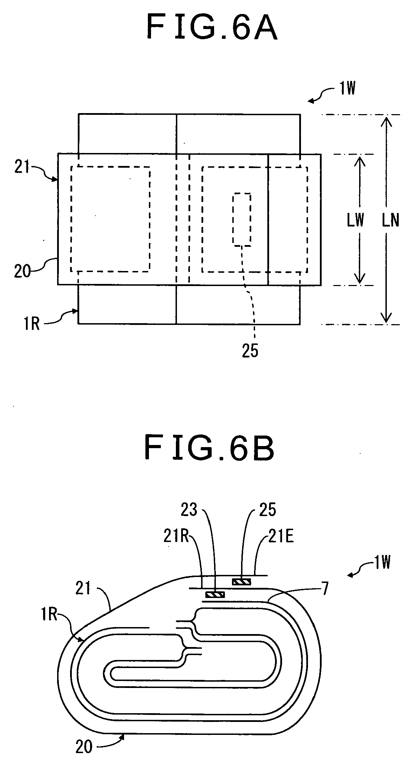

In the example shown in FIGS. 6A and 6B, the belt-shaped material 21 is provided to extend in the longitudinal direction of the napkin 1. Further, the belt-shaped material 21 is provided as a single piece. In this case, one end 21E of the belt-shaped material 21 in the longitudinal direction is superposed over the remaining part 21R of the belt-shaped material 21, for example, the other end in the longitudinal direction, and attached there by an adhesive strip 25.

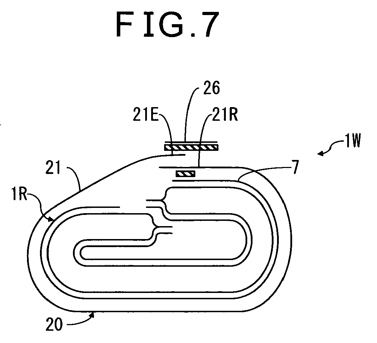

Alternatively, as shown in FIG. 7, the end 21E of the belt-shaped material 21 in the longitudinal direction may be attached to the remaining part 21R by adhesive tape 26.

In the example shown in FIGS. 6A and 6B and FIG. 7, if the wrapping material 20 and the peeloff sheet 7 are inseparably attached, it is possible to perform the operation of removing the wrapping material 20 from the napkin 1 and the operation of removing the peeloff sheet 7 from the absorbent body 2 consecutively, therefore it is possible to streamline the attachment of the napkin 1.

Note that, various shapes of the ready-to-wrap napkin 1 may be considered. For example, it is also possible to fold the napkin 1 so that the back part 1B is superposed over the front part 1F. Alternatively, it is possible to fold the napkin 1 along a folding region which extends in the longitudinal direction of the napkin 1. Further, the number of the folding regions may be set in any way.

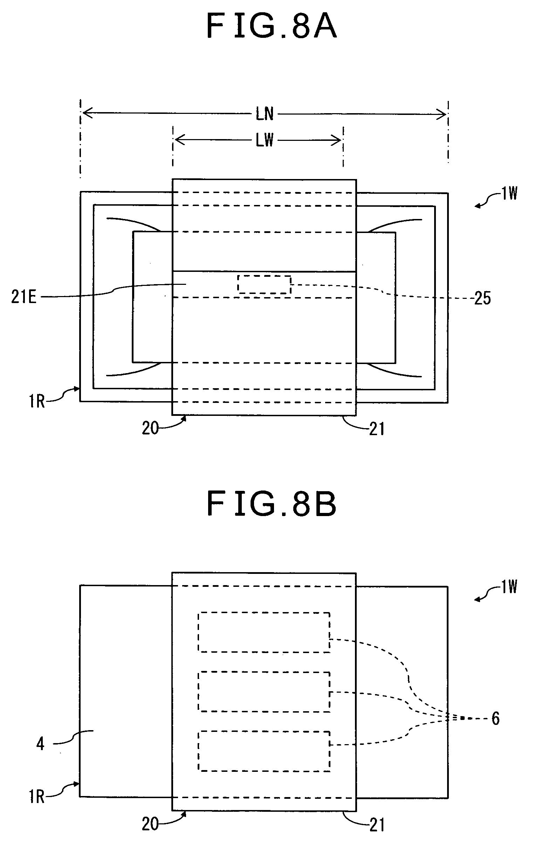

In the example shown in FIGS. 8A and 8B, no folding region is provided, therefore a ready-to-wrap napkin 1R is formed without the napkin 1 being folded.

By doing this, it is possible to streamline the production process of the individually wrapped product 1W. Further, the napkin 1 is not creased, so it is possible to prevent gaps forming between the napkin 1 and the wearer.

Further, in the example shown in FIGS. 8A and 8B, the wrapping material 20 is attached to the ready-to-wrap napkin 1R in a peelable fashion through the adhesive strips 6 which are provided at the outside surface of the non-skin contact side sheet 4. As a result, the wrapping material 20 can be kept from deviating in position and the peeloff sheet 7 can be omitted.

Here, the adhesive strips 6, as shown in FIG. 8B, are applied to the non-skin contact side sheet 4 in a pattern of three strips. Naturally, the adhesive strips 6 can be applied in any pattern.

In the above embodiments, at the center region 8C of the cover sheet 8, the partition lines 9 are formed so that four wings are defined. However, the number of the wings may be set in any way.

Further, the embodiments explained up to here may be combined with each other. That is, for example, the wrapping material 20 of FIGS. 6A and 6B may also be comprised of an upper piece and a lower piece, and the end 21E of the belt-shaped material 21 in the longitudinal direction in FIGS. 8A and 8B may be fixed in place by adhesive tape.

### Reference Signs List

- 1: sanitary napkin
- 1R: ready-to-wrap napkin
- 2: absorbent body
- 3: skin contact side sheet
- 4: non-skin contact side sheet
- 5: absorbent core
- 8: cover sheet
- 9: partition line
- 10F, 10B, 10L, 10R: wings
- 20: wrapping material
- 21: belt-shaped material

## Claims

1. An individually wrapped absorbent product provided with an absorbent body (2) which includes a liquid permeable skin contact side sheet (3), a liquid impermeable non-skin contact side sheet (4), and an absorbent core (5) which is arranged between these skin contact side sheet (3) and non-skin contact side sheet (4) and with a cover sheet (8) which covers the surface of the skin contact side sheet (3), which individually wrapped product is formed by wrapping an absorbent product in a wrapped state, defined as a "ready-to-wrap product", by a wrapping material, in said individually wrapped absorbent product,
said wrapping material (20) including a belt-shaped material (21) covering the ready-to-wrap product over the entire circumference of the ready-to-wrap product,
a width of said belt-shaped material (21) in a width direction of the belt-shaped material being shorter than a length of the ready-to-wrap product in the width direction of the belt-shaped material (21),
wherein the cover sheet (8) is comprised of a plastic film having a drapeability, by the cantilever method, of 20 mm to 100 mm,
wherein a peripheral edge region of the cover sheet (8) is bonded to said absorbent body (2), a center region of the cover sheet (8) has partition lines (9) comprised of weakened lines or cut lines by which at least one wing for affixing the absorbent body (2) to clothing is defined, and, at the time of use, the wing is folded and extended at the folded region and affixed to the clothing, and wherein
said partition lines (9) are formed so that said wings includes at least one of a front wing (10F) and a back wing (10B) which are to be folded at folded regions along front and back end edges of said absorbent body (2) and extended in the front and back directions,
said ready-to-wrap product is formed by folding said absorbent product along at least one folding region which extends in a width direction of said absorbent product so that said cover sheet (8) is positioned at the inside, and
said belt-shaped material (21) is provided at said ready-to-wrap product so as to extend cutting across the side edges of said absorbent product and across ends of said partition lines.

2. An individually wrapped absorbent product as set forth in claim 1, wherein said ready-to-wrap product is formed by folding said absorbent product along at least one folding region which extends in a width direction of said absorbent product so that said cover sheet (8) is positioned at the inside.

3. An individually wrapped absorbent product as set forth in any one of claims 1 to 2, wherein said belt-shaped material (21) is provided at said ready-to-wrap product so as to extend cutting across the side edges of said absorbent product.

4. An individually wrapped absorbent product as set forth in claim 2, wherein
said absorbent product is folded so that one end of said absorbent product in the longitudinal direction is superposed over a remaining part of said absorbent product, and
said belt-shaped material (21) is provided at said ready-to-wrap product so as to cut across side edges of said absorbent product and extend across one end said absorbent product in the longitudinal direction.

5. An individually wrapped absorbent product as set forth in any one of claims 1 to 4, wherein said wrapping material is attached to said ready-to-wrap product.

## Patentansprüche

1. Einzeln verpacktes saugfähiges Produkt mit einem saugfähigen Körper (2) versehen, der eine flüssigkeitsdurchlässige Hautkontaktseitenlage (3), eine flüssigkeitsundurchlässige Nicht-Hautkontaktseitenlage (4) und einen saugfähigen Kern (5) umfasst, der zwischen dieser Hautkontaktseitenlage (3) und dieser NichtHautkontaktseitenlage (4) mit einer Abdecklage (8), die die Oberfläche der Hautkontaktseitenlage (3) abdeckt, wobei das einzeln verpackte Produkt durch das Verpacken eines saugfähigen Produkts, definiert als ein "fertig-zum-Verpacken Produkt", durch ein Verpackungsmaterial in einen verpackten Zustand gebildet wird, in dem einzeln verpackten saugfähigen Produkt angeordnet ist,
wobei das Verpackungsmaterial (20) ein riemenförmiges Materials (21) umfasst, das das fertig-zum-Verpacken Produkt über den gesamten Umfang des fertig-zum-Verpacken Produkts abdeckt,
eine Breite des riemenförmigen Materials (21) in eine Breitenrichtung des riemenförmigen Materials kürzer ist als eine Länge des fertig-zum-Verpacken Produkts in die Breitenrichtung des riemenförmigen Materials (21),
wobei die Abdecklage (8) aus einem Plastikfolie gebildet ist, die eine Drapierfähigkeit von 20 mm bis 100 mm, nach dem Cantilever-Verfahren, hat,
wobei ein äußerer Kantenbereich der Abdecklage (8) an den saugfähigen Körper (2) geklebt ist, ein Mittelbereich der Abdecklage (8) Trennlinien (9) hat, gebildet aus geschwachten Linien oder geschnittenen Linien, durch die zumindest ein Flügel zum Befestigen des saugfähigen Körpers (2) an Kleidung gebildet wird, und der Flügel zum Zeitpunkt der Verwendung gefaltet und ausgestreckt an dem gefalteten Bereich und an der Kleidung befestigt ist, und wobei
die Trennlinien (9) so gebildet sind, dass die Flügel zumindest einen eines Vorderflügels (10F) und eines Hinterflügels (10B) umfasst, die an gefalteten Bereichen entlang von vorderen und hinteren Endkanten des saugfähigen Körpers (2) zu falten sind und sich in die Vorder- und Hinterrichtung erstrecken,
das fertig-zum-Verpacken Produkt durch Falten des saugfähigen Produkts entlang zumindest eines Faltbereichs, der sich in eine Breitenrichtung des saugfähigen Produkts erstreckt, gebildet wird, so dass die Abdecklage (8) auf der Innenseite liegt, und
das riemenförmige Material (21) an dem fertig-zum-Verpacken Produkt vorgesehen ist, so dass es sich über die Seitenkanten des saugfähigen Produkts und über Enden der Trennlinien schneidend erstreckt.

2. Einzeln verpacktes saugfähiges Produkt nach Anspruch 1, wobei das fertig-zum-Verpacken Produkt durch Falten des saugfähigen Produkts entlang zumindest einer Faltregion gebildet wird, welche sich in eine Breitenrichtung des saugfähigen Produkts erstreckt, so dass die Abdecklage (8) auf der Innenseite liegt.

3. Einzeln verpacktes saugfähiges Produkt nach Anspruch 1 oder 2, wobei das riemenförmige Material (21) an dem fertig-zum-Verpacken Produkt vorgesehen ist, so dass es sich über die Seitenkanten des saugfähigen Produkts schneidend erstreckt.

4. Einzeln verpacktes saugfähiges Produkt nach Anspruch 2, wobei
das saugfähige Produkt so gefaltet ist, dass ein Ende des saugfähigen Produkts in die Längsrichtung über einen verbleibenden Teil des saugfähigen Produkts überlagert ist, und
das riemenförmige Material (21) an dem fertig-zum-Verpacken Produkt vorgesehen ist, sodass es über die Seitenkanten des saugfähigen Produkts schneidet und sich über ein Ende des saugfähigen Produkts in die Längsrichtung erstreckt.

5. Einzeln verpacktes saugfähiges Produkt nach einem der Ansprüche 1 bis 4, wobei das Verpackungsmaterial an dem fertig-zum-Verpacken Produkt befestigt ist.

## Revendications

1. Produit absorbant à emballage individuel pourvu d'un corps absorbant (2) qui inclut une feuille perméable aux liquides côté de contact avec la peau (3), une feuille imperméable aux liquides côté de non-contact avec la peau (4) et un coeur absorbant (5) qui est agencé entre ces feuille côté de contact avec la peau (3) et feuille côté de non-contact avec la peau (4) et d'une feuille de couverture (8) qui couvre la surface de la feuille côté de contact avec la peau (3), lequel produit à emballage individuel est formé en emballant un produit absorbant dans un état emballé, défini comme « produit prêt à emballer », par un matériau d'emballage, dans ledit produit absorbant à emballage individuel,
ledit matériau d'emballage (20) incluant un matériau en forme de bande (21) couvrant le produit prêt à emballer sur toute la circonférence du produit prêt à emballer, une largeur dudit matériau en forme de bande (21) dans une direction de largeur du matériau en forme de bande étant plus courte qu'une longueur du produit prêt à emballer dans la direction de largeur du matériau en forme de bande (21),
dans lequel la feuille de couverture (8) se compose d'un film plastique ayant une aptitude au drapé, par le procédé en porte-à-faux, de 20 mm à 100 mm,
dans lequel une région de bord périphérique de la feuille de couverture (8) est liée audit corps absorbant (2), une région centrale de la feuille de couverture (8) comporte des lignes de séparation (9) composées de lignes affaiblies ou lignes de découpe par lesquelles au moins une aile pour fixer le corps absorbant (2) au vêtement est définie, et, au moment de l'utilisation, l'aile est pliée et étendue au niveau de la région pliée et fixée au vêtement, et dans lequel
lesdites lignes de séparation (9) sont formées de telle sorte que lesdites ailes incluent au moins une parmi une aile avant (10F) et une aile arrière (10B) qui doivent être pliées au niveau de régions pliées le long de bords d'extrémité avant et arrière dudit corps absorbant (2) et s'étendant dans les directions avant et arrière,
ledit produit prêt à emballer est formé en pliant ledit produit absorbant le long d'au moins une région de pliage qui s'étend dans une direction de largeur dudit produit absorbant de telle sorte que ladite feuille de couverture (8) est positionnée à l'intérieur, et
ledit matériau en forme de bande (21) est disposé au niveau dudit produit prêt à emballer de manière à étendre la découpe en travers des bords latéraux dudit produit absorbant et en travers desdites lignes de séparation.

2. Produit absorbant à emballage individuel selon la revendication 1, dans lequel ledit produit prêt à emballer est formé en pliant ledit produit absorbant le long d'au moins une région de pliage qui s'étend dans une direction de largeur dudit produit absorbant de telle sorte que ladite feuille de couverture (8) est positionnée à l'intérieur.

3. Produit absorbant à emballage individuel selon l'une quelconque des revendications 1 à 2, dans lequel ledit matériau en forme de bande (21) est disposé au niveau dudit produit prêt à emballer de manière à étendre la
découpe en travers des bords latéraux dudit produit absorbant.

4. Produit absorbant à emballage individuel selon la revendication 2, dans lequel
ledit produit prêt à emballer est plié de telle sorte qu'une extrémité dudit produit absorbant dans la direction longitudinale est superposée sur une partie restante dudit produit absorbant, et
ledit matériau en forme de bande (21) est disposé au niveau dudit produit prêt à emballer de manière à découper en travers des bords latéraux dudit produit absorbant et à s'étendre sur une extrémité dudit produit absorbant dans la direction longitudinale.

5. Produit absorbant à emballage individuel selon l'une quelconque des revendications 1 à 4, dans lequel ledit matériau d'emballage est attaché audit produit prêt à emballer.
